# EUROPEAN PATENT APPLICATION

(11) **EP 2 529 779 A1**
(43) Date of publication of application: **05.12.2012**
(21) Application number: 11736657.5
(22) Date of filing: 26.01.2011
(51) Int. Cl.: A61M 13/00, A61M 3/02, A61M 25/01

(54) **CATHETER FOR PERFORMING IMAGE-BASED COLONOSCOPIES**

(30) Priority: 29.01.2010 ES 201030120
(71) Applicant: Costovici, Nicolas Anthony, 17480 Roses (ES)
(72) Inventor: Costovici, Nicolas Anthony, 17480 Roses (ES)
(74) Representative: Gallego Jiménez, José Fernando
(86) International application number: PCT/ES2011/000018
(87) International publication number: WO 2011/092358

(57) **Abstract**

The invention relates to a catheter comprising a member (1) that can be inserted into a person's rectum, said member including a first rear opening (11) that communicates with a first passage (12) for discharging bodily fluids from an aperture (13) at the front end of the insertable member (1). The instertable member (1) includes an expandable ring element (14) communicating with a second rear opening (15) for the connection to an inflation device. The catheter includes a probe (2) disposed at the front end of a connection pipe (4), which pipe is housed in the first passage (12) and connected at the rear end thereof to a device (3) for supplying a pressurised liquid, said probe (2) including several holes (21) in the rear portion thereof for projecting the liquid backwards, cleaning the colon and pushing the

## Description

### Object of the invention.

The present invention relates to a catheter for performing image-based colonoscopies, more particularly a duct that permits the collection of body effluents and the gas insufflation for the distension of the colon.

### Background of the invention.

Currently catheters are used for performing image-based colonoscopies. A typical catheter comprises an insertable member into an individual cavity, in this case into the rectum, said member presenting a first insertable rear opening that communicates with a first passage for discharging bodily fluids and connectable to at least one device for supplying gas into the individual's body cavity through an opening at the front end of the insertable member. The insertable member is provided with an expandable ring element in proximity with the front end communicating through a second passage, with a second rear opening of the insertable member, for connection to an inflation device for the expandable ring element.

The gas insufflation operation in the colon allows for the distension of the colon.

When performing an image-based colonoscopy the patient should prepare one or two days before the exam by ingesting a liquid comprising a marker, contrasting agent and a laxative product, since the insertable member has a low effluents extraction capacity. These products mainly cause both the emptying of the colon in order to suitably perform the colonoscopy later and the marking of polyps, tumours and those points which are of particular interest for the diagnosis of possible diseases. This preparation stage is carried out by the patient at home and is very unpleasant, because it produces thorough faeces evacuation to remove all traces from the colon walls, allowing a proper examination and diagnosis. Furthermore, the amount of marker and contrasting agent to be used is significantly higher than strictly necessary, as it is diluted with the laxative product and is eliminated producing a low performance.

### Description of the invention.

The catheter for performing image-based colonoscopies, object of this invention, presents technical features intended to facilitate the quality of such examination and diagnosis and patient comfort. 1. - The catheter comprises a member that can be inserted into a person's rectum, for filling the colon with a distensing gas supplied by a gas insufflation device, said insertable member presenting a first rear opening that communicates with a first passage for discharging body effluents through an opening at the front end of the insertable member. The insertable member is provided with an expandable ring element in proximity with the front end communicating, through a second passage, with a second rear opening of the insertable member, for connection to an inflation device of said expandable ring element.

According to the invention, the catheter comprises a probe arranged at the front end of a of a connection pipe, which is housed in the first passage, and connected at the rear end to an outer device for supplying a pressurized liquid, said probe including several holes at the rear portion thereof for projecting the liquid backwards, cleaning the colon effluents and pushing the probe forwards to progress in the preparation of the colon for the subsequent image-based colonoscopy.

The insertable member presents a watertight passage at the first passage by which the connection pipe is inserted in a sliding form to facilitate the advance and retraction of the probe.

In this way it is not necessary for the patient to be prepared by ingesting the liquid containing the marker, contrasting agent and the laxative product, thus avoiding the inconvenience of continuing previous evacuations. The liquid is also applied locally just before the colonoscopy, allowing a reduction of the amount of liquid required and improving the quality of the diagnosis as the colon cleaning is very recent.

After cleaning with the probe and once all effluents have been evacuated and removed then the insufflation with gas for the colonoscopy may be carried out.

In a preferred embodiment, the probe is of a hollow spherical shape, since it is not as unpleasant when it moves inside the colon and enables a better backward projection of the liquid. In the event that the probe comes across a polyp obstructing its passage, it will simply stop without causing discomfort or injury as it is spherical.

In one embodiment, the connection pipe comprises at its rear section means for the collection and delivery of said connection pipe depending on the advance of the probe. For example, such a device is powered and electronically controlled by the device for supplying a pressurized liquid, making the entire process automatic.

In an alternative embodiment, the insertable member has been provided to comprise an additional passage between the front end opening and an additional outlet rear opening for controlling overpressure in the colon.

In a first embodiment the insufflation of gas in the colon is performed through the first passage. In an alternative embodiment, the connection pipe comprises, at its rear section, a branch with closing means, for example a clamp, connected to the device for insufflating gas for the introduction of said gas into the colon after cleaning, so that the first passage is permanently ready for the collection of remaining effluents.

### Description of figures.

In order to complement the description that is being carried out and with the purpose of facilitating the understanding of the characteristics of the invention, the present description is accompanied by a set of drawings wherein, by way of a nonlimiting example, the following has been represented:
- Figure 1 shows a plan view of the catheter according to the invention.
- Figure 2 shows a plan view of the catheter according to the invention, equipped with an overpressure control passage and opening.
- Figure 3 shows a perspective view of a detail of the probe and front end of the insertable member.
- Figure 4 shows a schematic of the application of the catheter associated with the apparatus for insufflating gas, for supplying liquid and for the aspiration of effluents.

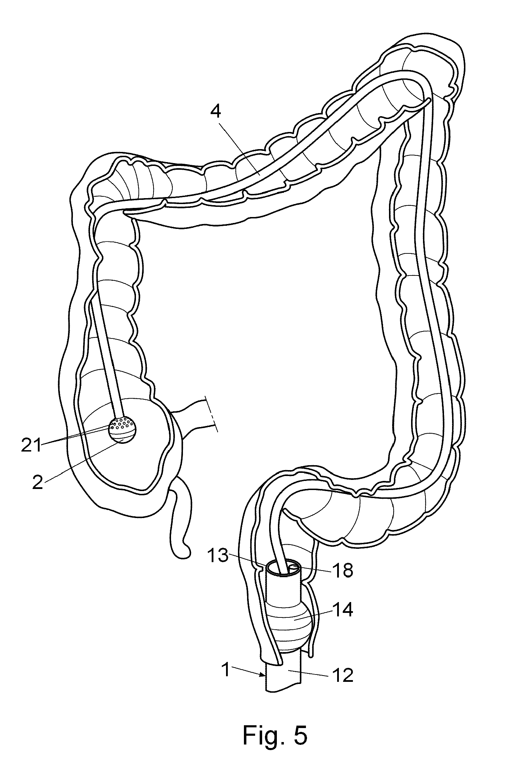

- Figure 5 shows a schematic of the application of the catheter in the colon and the cleaning procedure thereof.

### Preferred embodiment of the invention.

As can be seen in the figures referring to the catheter for performing image-based colonoscopies according to the present invention, it comprises an insertable member (1) having a first rear opening (11) that communicates with a first passage (12) for discharging bodily effluents through an aperture (13) at the front end of the insertable member (1), with the insertable member (1) being also provided with an expandable ring element (14) in proximity to said front end, and connected through a second passage (15) to a second rear opening (16) of the insertable member (1), for connection to an inflation device (not shown). The catheter comprises a probe (2) with a plurality of holes (21) at its rear portion for projecting liquid from a supply device (3) through a connection pipe (4) connected to the rear portion of this probe (2). Said connection pipe (4) is housed in the first passage (12), from a watertight passage (17) at the rear of the insertable member (1) to the opening (13) of the front end through which the probe (2) is ready to advance into the colon of the patient by proceeding to cleaning.

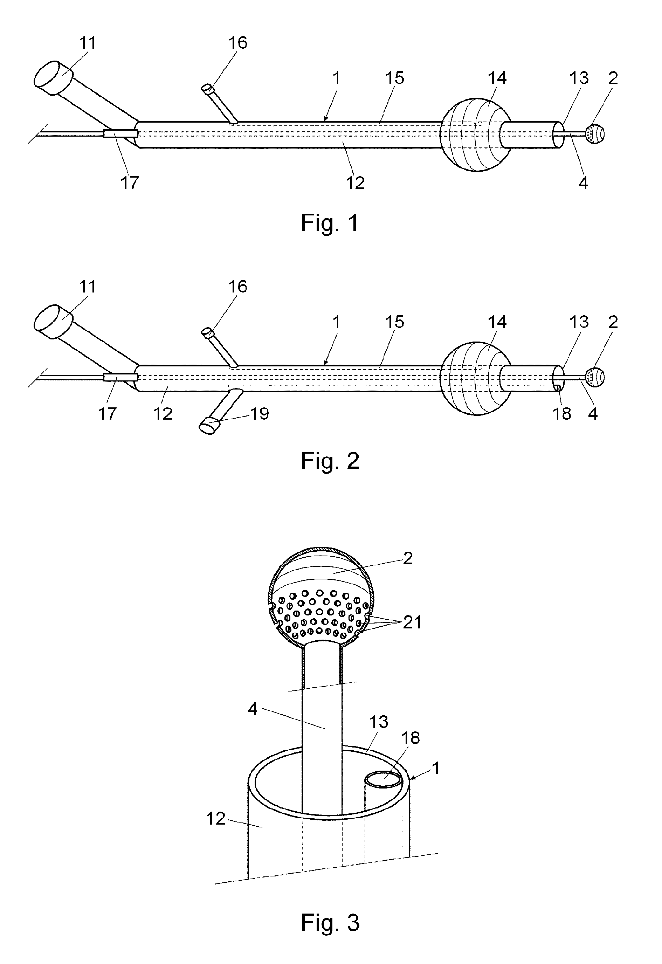

In this case, the probe (2) is spherical and hollow as shown in Figure 3, with holes (21) arranged at the rear half thereof, where the connection pipe (4) is connected.

Shown in Figure 2 is a variant of the catheter which presents an additional passage (18) between the opening (13) of the front end and an additional rear outlet opening (19) for controlling overpressure in the colon.

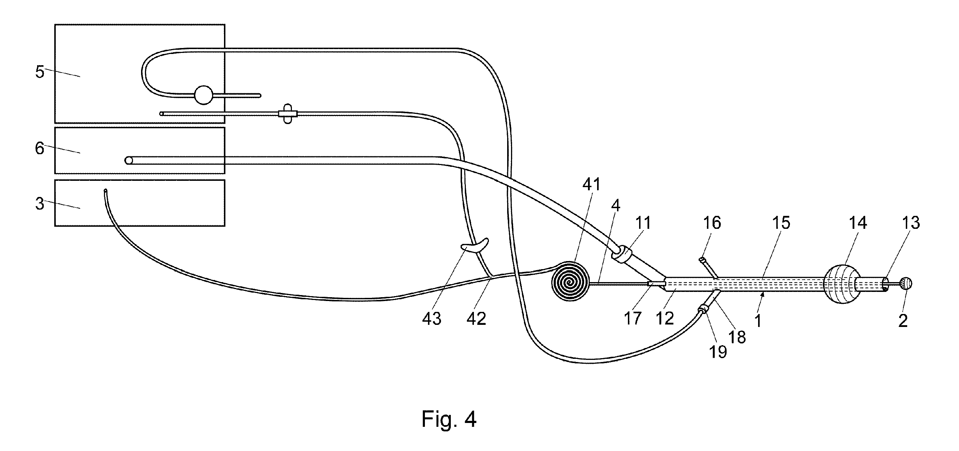

Shown in Figure 4 is a complete embodiment of the catheter, with the rear section of the connection pipe (4) having means (41) for collecting and supplying said connection pipe (4) to the insertable member (1), and, at a rear position, a branch (42) with a clamp as closing means (43) for an alternative section. This branch (42) is connected via the closure means (43) of the alternative section of the device (5) for insufflating gas for the colonoscopy.

Shown in this Figure 4 is how in a same machine the device (3) for supplying liquid, the device (5) for insufflating gas, and an aspirator (6) connected to the first opening (12) of the insertable member (1) for discharging effluents, are arranged.

Once the nature of the invention as well as an example of preferred assembly have been sufficiently described, it is stated for all pertinent purposes that the materials, form, size and arrangement of the elements described are susceptible to changes, provided these do not involve an alteration of the essential characteristics of the invention that are claimed subsequently.

## Claims

1. Catheter for performing image-based colonoscopies, of the type comprising a member (1) that can be inserted into a person's rectum for filling the colon with a distending gas supplied by a device (5) for insufflating gas, said insertable member (1) having a first rear opening (11) that communicates with a first passage (12) for discharging bodily effluents through an aperture (13) at the front end of said insertable member (1), the insertable member (1) being provided with an expandable ring element (14) in proximity to said front end, and connected through a second passage (15) to a second rear opening (16) of the insertable member (1), for connection to an inflation device of said expandable ring element (14), **characterized in that** it comprises a probe (2) disposed at the front end of a connection pipe (4), which pipe is housed in the first passage (12) and connected at the rear end thereof to an external device (3) for supplying a pressurized liquid, said probe (2) including several holes (21) at the rear portion thereof for projecting the liquid backwards, cleaning the existing effluent in the colon and pushing the probe (2) forwards to progress in the preparation of the colon for the subsequent image-based colonoscopy; and **in that** the insertable member (1) has a watertight passage (17) in the first passage (12) through which the connection pipe (4) is slidably introduced to facilitate the advance and retraction of the probe (2).

2. Catheter according to claim 1, **characterized in that** the probe (2) is of hollow spherical shape.

3. Catheter according to any of the preceding claims, **characterized in that** the connection pipe (4) comprises at its rear section means (41) for collecting and supplying said connection pipe (4) depending on the advance of the probe (2).

4. Catheter according to any of the preceding claims, **characterized in that** the insertable member (1) comprises an additional passage (18) between the opening (13) of the front end and an additional rear outlet opening (19) for controlling overpressure in the colon.

5. Catheter according to any of the preceding claims, **characterized in that** the connection pipe (4) comprises at its rear section a branch (42) with closing means (43), connected to the device (5) for insufflating gas for introducing said gas into the colon after having performed the cleaning.
